# EUROPÄISCHE PATENTSCHRIFT

(11) **EP 2 410 047 B1**
(45) Veröffentlichungstag und Bekanntmachung des Hinweises auf die Patenterteilung: **01.06.2016**
(21) Anmeldenummer: 11177932.8
(22) Anmeldetag: 07.12.2007
(51) Int. Cl.: C12N 9/02, C12N 9/04, C12P 33/00

(54) **Oxidoreduktase und deren Verwendung zur Reduktion von Secodionderivaten**
Oxidoreductase and its use for the reduction of secodione derivatives
Oxydo-réductase et son utilisation dans la réduction de dérivés de sécodione

(30) Priorität: 07.12.2006 AT 20272006
(43) Veröffentlichungstag der Anmeldung: 25.01.2012
(62) Teilanmeldung aus: 07856445.7
(73) Patentinhaber: Cambrex IEP GmbH, 65203 Wiesbaden (DE)
(72) Erfinder: Gupta, Antje, 65207 Wiesbaden (DE); Tschentscher, Anke, 65347 Eltville - Hattenheim (DE); Dupont, Maria, 50354 Hürth (DE)
(74) Vertreter: Plate, Jürgen

(56) Entgegenhaltungen:
- EP-A1- 1 152 054
- WO-A-2006/087235
- WO-A1-2007/073875
- DE-A1-102005 044 736
- SZENTIRMAI, A. ET AL.: "Properties of hydroxysteroid oxidoreductase isolated from yeast", ACTA MICROBIOLOGICA ACADEMIAE SCIENTIARUM HUNGARICAE, Bd. 22, Nr. 4, 1975, Seiten 463-470, XP008095188,

## Beschreibung

Die vorliegende Erfindung betrifft eine Oxidoreduktase für die enantioselektive enzymatische Reduktion von Secodionderivaten der allgemeinen Formel I, wobei das Secodionderivat mit einer Oxidoreduktase/Dehydrogenase in Gegenwart von NADH oder NADPH als Cofactor reduziert wird.

Die industrielle Darstellung von Steroidhormonen erfolgt auf zwei, voneinander unabhängigen Wegen, nämlich zum einen ausgehend von natürlich vorkommenden Steroidverbindungen aus pflanzlichen Quellen und zum anderen totalsynthetisch durch enantioselektive Synthese aus prochiralen Vorstufen. Von diesen beiden Wegen gewinnt die Steroidtotalsynthese zunehmend an Bedeutung, zumal diese auch die Einfuhrung von Strukturelementen erlaubt, die in natürlich vorkommenden Steroiden nicht enthalten sind.

Schlüsselbausteine der Totalsynthese von enantiomerenreinen Steroiden sind dabei Verbindungen der allgemeinen Formel I, welche auch als Secosteroide, 8,14-seco-gona-tetraen-14,17-dione oder Secodione bezeichnet werden. Spezielle Vertreter dieser Gruppe sind zum Beispiel die Verbindungen Methylsecodion (Formel II, 13-Methyl-3-methoxy-8,14-seco-gona-1,3,5(10),9(11)-tetraen-14,17-dion) und Ethylsecodion (Formel III, 13-Ethyl-3-methoxy-8,14-seco-gona-1,3,5(10),9(11)-tetraen-14,17-dion), aus denen beispielsweise die pharmakologisch wirksamen Verbindungen Ethinylestradiol (Formel IV) und Norgestrel (Formel V) hergestellt werden können.

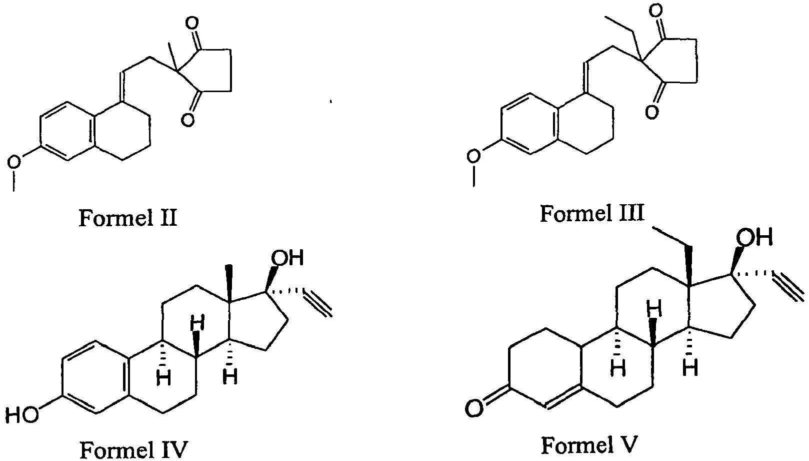

Schlüsselschritt bei der Darstellung enantiomerenreiner Steroidverbindungen ist dabei die Überführung der Verbindung der Formel I (z.B. II und III) in eine optisch aktive Verbindung mit vorgebildetem asymmetrischem C-13 durch enantioselektive Reduktion einer der Ketogruppen zur Hydroxygruppe. Die resultierenden optisch aktiven Hydroxysecosteroidverbindungen (Secole, Formeln VI bis IX) können anschließend durch Zyklisierung unter Erhalt der Chiralität zu chiralen Steroidverbindungen weiterverarbeitet werden.

Durch enantioselektive Reduktion einer Ketogruppe der Verbindung der Formel I können theoretisch vier optisch aktive Verbindungen gebildet werden (Formeln VI bis IX).

| | | | |
|---|---|---|---|
| 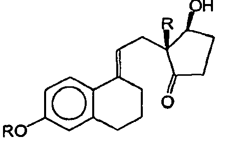 | 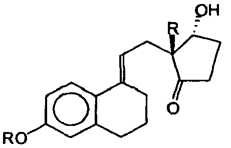 | 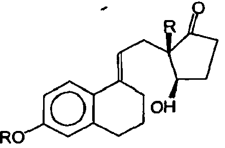 | 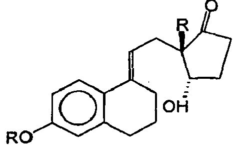 |
| Formel VI (17-beta-OH) | Formel VII (17-alpha-OH) | Formel VIII (14-beta-OH) | Formel IX (14-alpha-OH) |

Wirtschaftlich von besonderem Interesse sind dabei Verbindungen der Formel VI, bei denen die Hydroxygruppe an der Position 17 die beta-Konfiguration aufweist, da diese zu Derivaten des natürlichen Östron führen. Solche Verbindungen werden auch als 17-beta-Hydroxysecosteroide bezeichnet.

Die stereoselektive Reduktion von Secodionderivaten der allgemeinen Formel I mit Hilfe verschiedener Mikroorganismen wurde besonders intensiv in den 1960er und 1970er Jahren untersucht. Dabei konnte gezeigt werden, dass verschiedene Hefestämme der Gattung Candida, Debaryomyces, Kloeckera, Pichia, Cryptococcus, Rhodotorula, Torulopsis und Hansenula in der Lage sind Secodione zu den verschiedenen Hydroxyverbindungen zu reduzieren (US 3616226, US 1252524, US 3616225).

Insbesondere Hefen der Gattung Saccharomyces wie beispielsweise S. uvarum können vorteilhaft eingesetzt werden, um beispielsweise die entsprechenden 17-beta-Hydroxysecosteroide darzustellen (Kosmol et al; Liebigs Ann. Chem. 701,199 (1967)). Andere Hefestämme wie beispielsweise Saccharomyces drosophilarum reduzieren Secodion bevorzugt zum korrespondierenden 14-alpha-Hydroxysecosteroid (Acta microbiol. Acad. Sci. hung. 22,463-471 (1975)). Die Bildung des 14-alpha-Hydroxysecosteroids wird des weiteren auch durch Reduktion von Secodion mittels Bacillus thuringiensis beschrieben (Kosmol et al.; Liebigs Ann. Chem. 701,199 (1967)).

Gestagen- und Östrogenwirkstoffe finden weltweit breite Anwendung als Kontrazeptiva und in der Hormonersatztherapie. Bis heute sind die meisten Synthesen von Östrogen- und Gestagenderivaten auf dem oben beschriebenen Reaktionsprinzip aufgebaut, dessen Schlüsselschritt die enantioselektive Reduktion von Secodionen zu den entsprechenden 17-beta-Hydroxysecosteroiden darstellt.

Die stereoselektive Reduktion der Secodionderivate wird dabei bis heute als Ganzzellbiotransformation mittels verschiedener Hefestämme der Gattung Pichia oder Saccharomyces durchgeführt. Diese Verfahren besitzen jedoch den Nachteil, dass nur sehr geringe Substratkonzentrationen von weit unter 1% (i.d.R. 1 bis 5 g/1) realisierbar sind (US 3697379; Current Science, Feb.5 (1984), Vol 53. No. 3, Seite 124; Indian Journal of Experimental Biology, Vol.27, August 1989, Seite 742-743). Dadurch gestaltet sich insbesondere die Aufarbeitung und Isolierung des Reaktionsproduktes aus großen Volumina sowie die Abtrennung großer Mengen von Biomasse sehr aufwendig.

Nach Wissen der Erfinder wurden die an der Reduktion beteiligten Enzyme bislang nicht isoliert, identifiziert und beschrieben. Ebenso wurden noch keine DNA-Sequenzen identifiziert, die für Oxidoreduktasen codieren, mit denen die Reduktion von Secodionderivaten erreicht werden kann.

Die Erfindung stellt sich daher die Aufgabe, eine Oxidoreduktase bereitzustellen, mit der Secodionderivate der allgemeinen Formel I, insbesondere solche der Formel II und III, enantioselektiv reduziert werden können. Unter anderem soll hierdurch auch die Herstellung der entsprechenden 17-beta-Hydroxysecosteroide möglich sein.

Der Schutzumfang der Erfindung wird durch die Ansprüche 1 bis 3 bestimmt. In einem ersten Aspekt wird diese Aufgabe erfindungsgemäß durch eine Oxidoreduktase für die enantioselektive enzymatische Reduktion von Secodionderivaten der allgemeinen Formel I gelöst,

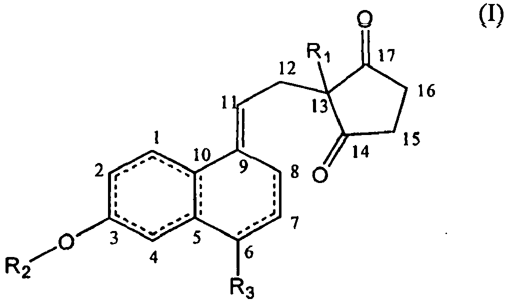

EP 1 152 054 B1 offenbart ein Polynukleotid, ein von dem Polynukleotid codiertes Polypeptid und ein Verfahren zur asymmetrischen Reduktion von tert-Butyl-(S)-6-chloro-5-hydroxy-3-oxohexanoat zu tert-Butyl-(3R, 5S)-6-chloro-3,5-dihydroxyhexanoat, wobei die Reduktionsreaktion mittels des Polypeptids katalysiert wird. Das Polynukleotid und das Polypeptid sind von Mikroorganismen der Gattung Candida, insbesondere der Spezies Candida magnoliae IFO 0705 ableitbar. worin die Ringstrukturen kein, ein oder mehrere Heteroatome umfassen,
R₁ Wasserstoff oder eine C₁-C₄-Alkylgruppe ist,
R₂ Wasserstoff, eine C₁-C₈-Alkylgruppe oder eine OH- Schutzgruppe, wie ein Ester, ist,
R₃ Wasserstoff, eine Methylgruppe oder ein Halogenid ist,
das Strukturelement 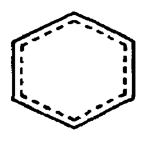 einen Benzolring oder einen C₆-Ring mit 0, 1 oder 2 C-C-Doppelbindungen repräsentiert, an den Positionen 6/7 oder 7/8 gegebenenfalls eine Doppelbindung enthalten ist und der Kohlenstoff an den Positionen 1, 2, 4, 5, 6, 7, 8, 9, 11, 12 und 16 unabhängig mit Wasserstoff, einer C₁-C₄-Alkylgruppe, einem Halogenid oder einer Phenylgruppe substituiert ist,
wobei die Oxidoreduktase/Dehydrogenase das Secodionderivat in Gegenwart von NADH oder NADPH als Cofactor reduziert.

Das Secodionderivat wird in einer Konzentration von >10 g/l im Reaktionsansatz eingesetzt und der durch die Oxidoreduktase/Dehydrogenase gebildete oxidierte Cofactor NAD oder NADP wird kontinuierlich regeneriert.

Dieses Verfahren stellt eine wesentliche Verbesserung der enantioselektiven enzymatischen Reduktion von Secodionderivaten gegenüber dem Stand der Technik dar. Die erfindungsgemäße Oxidoreduktase ermöglicht die Reduktion von Secodionderivaten zu den verschiedenen korrespondierenden Hydroxysecosteroiden mit freien Enzymen in Konzentrationsbereichen, die weit über die im Stand der Technik Beschriebenen hinausgehen. In einem zweiten Aspekt wird eine erfindungsgemäße Oxidoreduktase für die enantioselektive enzymatische Reduktion von Secodionderivaten der allgemeinen Formel I eingesetzt, wobei das Secodionderivat mit einer Oxidoreduktase/Dehydrogenase in Gegenwart von NADH oder NADPH als Cofactor reduziert wird, und die Oxidoreduktase/Dehydrogenase
a) eine Aminosäuresequenz aufweist, bei welcher mindestens 80% der Aminosäuren mit der Aminosäuresequenz SEQ ID NO:3 identisch sind oder
b) von der Nukleinsäuresequenz SEQ ID NO:8 codiert wird und von einer Nukleinsäuresequenz codiert wird, die mit SEQ ID NO:8 unter stringenten Bedingungen hybridisiert, wobei die stringenten Bedingungen die Hybridisierung in 0.7-1 M NaCl-Lösung bei 60°C umfassen.

Von den Erfindern wurden Oxidoreduktasen identifiziert, die in der Lage sind, Secodionderivate zu Hydroxysecosteroiden zu reduzieren, und die rekombinant industriell hergestellt werden können. Durch die erfindungsgemäße Oxidoreduktase können wesentlich höhere Substratkonzentrationen erzielt werden als bei den aktuell verwendeten Ganzzellverfahren.

In dem Verfahren kann die Oxidoreduktase mit der Sequenz SEQ ID NO:3 bzw. ein von diesem Polypeptid ableitbares Polypeptid entweder vollständig gereinigt, teilweise gereinigt oder als Zellen, enthaltend das Polypeptid SEQ ID NO:3 eingesetzt werden. Die eingesetzten Zellen können dabei nativ, permeabilisiert oder lysiert vorliegen. Bevorzugt werden die Oxidoreduktasen bzw. davon ableitbare Derivate in einem geeigneten Wirtsorganismus, wie beispielsweise *Escherichia coli,* überexprimiert und das rekombinante Polypeptid zur Reduktion der Secodionderivate der allgemeinen Formel I eingesetzt.

Eine nicht erfindungsgemäße DNA-Sequenz SEQ ID NO:6, die für ein Polypeptid mit der SEQ ID NO:1 codiert, ist beispielsweise aus dem Genom des Organismus *Chloroflexus aurantiacus* DSM 635 erhältlich.

Eine nicht erfindungsgemäße DNA-Sequenz SEQ ID NO:7, die für ein Polypeptid mit der SEQ ID NO:2 codiert, ist beispielsweise aus dem Genom des Organismus *Rubrobacter xylanophilus* DSM 9941 erhältlich.

Eine DNA-Sequenz SEQ ID NO:8, die für ein Polypeptid mit der SEQ ID NO:3 codiert, ist erhältlich aus einer Hefe *Candida magnoliae* CBS 6396.

Nicht erfindungsgemäße Oxidoreduktasen der SEQ ID NO:4 und SEQ ID NO:5 sind beispielsweise erhältlich durch Homologiescreening aus *Candida magnoliae* DSMZ 70638. Unter einer Nukleinsäuresequenz, welche beispielsweise mit der SEQ ID NO:6 unter stringenten Bedingungen hybridisiert, versteht man ein Polynukleotid, welches mittels der Kolonie-Hybridisierungsmethode, der Plaque-Hybridisierungsmethode, der Southern-Hybridisierungsmethode oder Vergleichbarem unter Verwendung der SEQ ID NO:6 oder Teilsequenzen von SEQ ID NO:6 als DNA-Sonde identifiziert werden kann. Zu diesem Zweck wird das an einem Filter immobilisierte Polynukleotid beispielsweise mit der SEQ ID NO:6 in einer 0,7-1 M NaCl-Lösung bei 60°C hybridisiert. Die Hybridisierung wird, wie z.B. in Molecular Cloning, A Laboratory Manual, Second Edition (Cold Spring Harbor Laboratory Press, 1989) oder ähnlichen Publikationen beschrieben, durchgeführt.

Anschließend wird der Filter mit 0,1 bis 2-facher SSC-Lösung bei 65°C gewaschen, wobei unter 1-facher SSC-Lösung ein Gemisch, bestehend aus 150 mM NaCl und 15 mM Natriumcitrat, verstanden wird.

Ein Polynukleotid, das mit den Polynukleotiden SEQ ID NO:6, SEQ ID NO:7; SEQ ID NO:8, SEQ ID NO:9 oder SEQ ID NO:10 aus der Sequenzliste unter oben genannten stringenten Bedingungen hybridisiert, weist zumindest 80% Sequenzidentität mit den

Polynukleotidsequenzen SEQ ID NO:6, SEQ ID NO:7, SEQ ID NO:8, SEQ ID NO:9 oder SEQ ID NO:10, vorzugsweise zumindest 95% Identität auf.

In einem weiteren Aspekt wird in einem Verfahren zur enantioselektiven enzymatischen Reduktion von Secodionderivaten der allgemeinen Formel I das Secodionderivat mit einer Oxidoreduktase/Dehydrogenase in Gegenwart von NADH oder NADPH als Cofactor reduziert, wobei die Oxidoreduktase/Dehydrogenase eine Länge von 230 bis 260 Aminosäuren aufweist und eine oder mehrere der Partialsequenzen, ausgewählt aus der Gruppe bestehend aus [den Sequenzen SEQ ID NO: 18 bis SEQ ID NO:42]
nalvtgasrgig, nalvtggsrgig, nalitggsrgig, nalitgasrgig, nalitggsrgmg, halvtgasrgig,
gysvtla, gynvtla, gysvtlv, gynvtlv,
fkgaplpa, fkaaplpa,
fvsnag, ffsnag, fvcnag, fvanag,
spialtkal, spvaltkti, spialtktl, spvamtkal, sqialtkal,
avysask, avysatk,
pikgwi und pisgwi, umfasst.

In den Verfahren wird als Co-Faktor NADH oder NADPH eingesetzt. Unter dem Begriff "NADP" wird Nicotinamid-adenin-dinucleotid-phosphat, unter "NADPH" reduziertes Nicotinamid-adenin-dinucleotid-phosphat verstanden. Der Begriff "NAD" bedeutet Nicotinamid-adenin-dinucleotid, der Begriff "NADH" reduziertes Nicotinamid-adenin-dinucleotid.

Gemäß einer Ausführungsform des Verfahrens, bei dem das Secodionderivat in einer Konzentration von >10 g/l im Reaktionsansatz eingesetzt wird und der durch die Oxidoreduktase/Dehydrogenase gebildete oxidierte Cofactor NAD oder NADP kontinuierlich regeneriert wird, weist die Oxidoreduktase/Dehydrogenase
a) eine Aminosäuresequenz auf, bei welcher mindestens 80% der Aminosäuren mit jenen der Aminosäuresequenz SEQ ID NO:3 identisch sind, oder
b) wird die Oxidoreduktase/Dehydrogenase von der Nukleinsäuresequenz SEQ ID NO:8 codiert.

Gemäß einer anderen Ausführungsform des Verfahrens, bei dem das Secodionderivat in einer Konzentration von >10 g/l im Reaktionsansatz eingesetzt wird und der durch die Oxidoreduktase/Dehydrogenase gebildete oxidierte Cofactor NAD oder NADP kontinuierlich regeneriert wird, weist die Oxidoreduktase/Dehydrogenase eine Länge von 230 bis 260 Aminosäuren auf und umfasst eine oder mehrere der Partialsequenzen, ausgewählt aus der Gruppe bestehend aus [den Sequenzen SEQ ID NO: 18 bis SEQ ID N0:42] nalvtgasrgig, nalvtggsrgig, nalitggsrgig, nalitgasrgig, nalitggsrgmg, halvtgasrgig, gysvtla, gynvtla, gysvtlv, gynvtlv, fkgaplpa, fkaaplpa, fvsnag, ffsnag, fvcnag, fvanag, spialtkal, spvaltkti, spialtktl, spvamtkal, sqialtkal, avysask, avysatk, pikgwi und pisgwi. Vorzugsweise wird bei den Verfahren gemäß dem zweiten und dritten Aspekt der durch die Oxidoreduktase/Dehydrogenase gebildete oxidierte Cofactor NAD oder NADP kontinuierlich regeneriert.

Gemäß einer Ausführungsform der Verfahren wird der oxidierte Cofactor NAD oder NADP durch Oxidation eines Alkohols regeneriert.

Als Cosubstrat werden dabei bevorzugt primäre und sekundäre Alkohole, wie Ethanol, 2-Propanol, 2-Butanol, 2-Pentanol, 3-Pentanol, 4-Methyl-2-pentanol, 2-Hexanol, 2-Heptanol, 2-Octanol oder Cyclohexanol eingesetzt. Der Anteil des Cosubstrates für die Regenerierung kann 5 bis 95 Vol%, bezogen auf das Gesamtvolumen, betragen.

Vorzugsweise wird zur Cofaktorregenerierung ein sekundärer Alkohol mit der allgemeinen Formel R_{X}R_{Y}CHOH verwendet wird, wobei R_{X} und R_{Y} unabhängig voneinander Wasserstoff, eine verzweigte oder unverzweigte C₁-C₈-Alkylgruppe sind und C_{insgesamt} ≥ 3.

Gemäß einer anderen Ausführungsform der Verfahren wird zur Regenerierung des Cofaktors zusätzlich eine Oxidoreduktase/ Dehydrogenase zugesetzt.

Geeignete NADH-abhängige Alkoholdehydrogenasen sind beispielsweise erhältlich aus Bäckerhefe, aus *Candida parapsilosis* (CPCR) (US 5,523,223 und US 5,763,236, Enzyme Microb. Technol., 1993, 15(11):950-8), *Pichia capsulata* (DE 10327454.4), aus *Rhodococcus erythropolis* (RECR) (US 5,523,223), *Norcardia fusca* (Biosci. Biotechnol. Biochem., 63(10), 1999, S. 1721-1729; Appl. Microbiol. Biotechnol,. 2003, 62(4):380-6; Epub 2003, Apr. 26) oder *Rhodococcus ruber* (J. Org. Chem., 2003, 68(2):402-6). Geeignete Cosubstrate für diese Alkoholdehydrogenasen sind beispielsweise die bereits genannten sekundären Alkohole wie 2-Propanol (Isopropanol), 2-Butanol, 2-Pentanol, 4-Methyl-2-pentanol, 2-Octanol oder Cyclohexanol.

Geeignete sekundäre Alkoholdehydrogenasen zur Regenerierung des NADPH sind beispielsweise solche, wie beschrieben und isoliert aus Organismen der Ordnung Lactobacillales, z.B. *Lactobacillus kefir* (US 5,200,335), *Lactobacillus brevis* (DE 19610984 A1; Acta Crystallogr. D. Biol. Crystallogr. 2000 Dec; 56 Pt 12:1696-8), *Lactobacillus minor* (DE 10119274), *Leuconostoc carnosum* (A 1261/2005, Kl. C12N) oder solche, wie beschrieben aus *Thermoanerobium brockii, Thermoanerobium ethanolicus* oder *Clostridium beijerinckii.*

Zur Cofactorregenerierung können prinzipiell aber auch andere enzymatische Systeme verwendet werden. Beispielsweise kann die Cofactorregenerierung mittels NAD- oder NADP-abhängiger Formiat-Dehydrogenase (Tishkov et al., J. Biotechnol. Bioeng. [1999] 64, 187-193, Pilot-scale production and isolation of recombinant NAD and NADP specific formate dehydrogenase) durchgeführt werden. Geeignete Cosubstrate der Formiat-Dehydrogenase sind beispielsweise Salze der Ameisensäure, wie Ammoniumformiat, Natriumformiat oder Calciumformiat.

Der in den Verfahren erreichte TTN (total turn over number = mol reduzierte Secodionverbindung / mol eingesetzter Cofactor) liegt in der Regel im Bereich von 10² bis 10⁵, vorzugsweise beträgt er jedoch ≥10³.

Gemäß einer Ausführungsform werden die Verfahren in einem wässrigen organischen Zweiphasensystem durchgeführt.

Demgemäß erfolgt die Umsetzung des Secodionderivates in einem Zweiphasensystem, enthaltend beispielsweise einen 2-Alkohol zur Cofactorregenerierung, eine Oxidoreduktase, Wasser, Cofaktor und die Secodionverbindung. Es können aber noch zusätzliche organische Lösungsmittel enthalten sein, die nicht an der Cofactorregenerierung beteiligt sind, d.h. keine oxidierbare Hydroxygruppen enthalten. Vorzugsweise werden als zusätzliche organische Lösungsmittel Diethylether, tertiär-Butylmethylether, Diisopropylether, Dibutylether, Ethylacetat, Butylacetat, Heptan, Hexan, Toluol, Dichlormethan, Cyclohexan oder Gemische davon eingesetzt.

Der Anteil der nicht wassermischbaren organischen Komponenten des Zweiphasensystems kann dabei von 10% bis 90%, bevorzugt von 20% bis 80%, bezogen auf das Gesamtvolumen des Reaktionsansatzes, betragen. Der wässrige Anteil kann von 90% bis 10%, bevorzugt von 80% bis 20%, bezogen auf das Gesamtvolumen des Reaktionsansatzes, betragen.

Dem Wasser kann auch ein Puffer zugesetzt werden, beispielsweise Kaliumphosphat-, Tris/HCl-, Glycin- oder Triethanolamin-Puffer mit einem pH-Wert von 5 bis 10, vorzugsweise von 6 bis 9. Der Puffer kann zusätzlich noch Ionen zur Stabilisierung oder Aktivierung beider Enzyme enthalten, beispielsweise Magnesiumionen oder Zinkionen.

Des weiteren können in den Verfahren noch weitere Zusätze zur Stabilisierung der verwendeten Enzyme eingesetzt werden, beispielsweise Glycerin, Sorbitol, 1,4-DL-Dithiothreit (DTT) oder Dimethylsulfoxid (DMSO).

Die Konzentration des Cofaktors NAD(P)H, bezogen auf die wässrige Phase, beträgt von 0,001 mM bis 10 mM, insbesondere von 0,01 mM bis 1,0 mM. Die Temperatur kann in Abhängigkeit von den speziellen Eigenschaften der eingesetzten Enzyme von 10°C bis 70°C betragen, bevorzugt von 20°C bis 35°C.

Die zu reduzierenden Secodionderivate sind in der Regel schwer wasserlöslich. Das Substrat kann daher während der Reaktion vollständig oder auch unvollständig gelöst vorliegen. Ist das Substrat im Reaktionsgemisch nicht vollständig gelöst, so liegt ein Teil des Substrates in fester Form vor und kann so eine dritte feste Phase bilden. Das Reaktionsgemisch kann während der Umsetzung auch zeitweise eine Emulsion bilden.

Das Secodionderivat der allgemeinen Formel I wird in den Verfahren vorzugsweise in einer Menge von 10g/l bis 500 g/1, bevorzugt von 25 g/l bis 300 g/1, besonders bevorzugt von 50g/l bis 200g/1, bezogen auf das Gesamtvolumen, im Reaktionsansatz eingesetzt.

Bevorzugte Ausführungsformen der Erfindung sind ferner dadurch gekennzeichnet, dass als Secodionderivat 13-Ethyl-3-methoxy-8,14-seco-gona-1,3,5(10),9(11)-tetraen-14,17-dion (Ethylsecodion - Formel III) oder 13-Methyl-3-methoxy-8,14-seco-gona-1,3,5(10),9(11)-tetraen-14,17-dion (Methylsecodion - Formel II) eingesetzt wird.

Die Verfahren werden beispielsweise in einem Reaktionsgefäß aus Glas oder Metall durchgeführt. Dazu werden die Komponenten einzeln in das Reaktionsgefäß überführt und unter einer Atmosphäre von beispielsweise Stickstoff oder Luft gerührt. Je nach eingesetzter Secodionverbindung und Oxidoreduktase beträgt die Reaktionszeit von einer Stunde bis 7 Tage, insbesondere von 2 Stunden bis 48 Stunden. In dieser Zeit wird die Secodionverbindung zu mindestens 50 % zur korrespondierenden Hydroxysecosteroidverbindung reduziert.

Die vorliegende Erfindung wird nachfolgend anhand von Beispielen näher erläutert.

### Beispiel 1 (nicht erfindungsgemäß)

### Klonierung einer Oxidoreduktase aus Chloroflexus auratiacus DSM 635

### A) Anzucht von Chloroflexus auratiacus DSM 635

Zellen von *Chloroflexus auratiacus* DSM 635 wurden in folgendem Medium (pH 8,2) bei 48°C in einem Bakterien-Inkubator bei Licht kultiviert: 0,1 % Hefeextrakt, 0,1 % Glycyl-Glycin, 0,01 % Na₂HPO₄ x 2 H₂O 0,01 % MgSO₄ x 7 H20, 0,01 % KNO₃, 0,05 % NaNO₃, 0,01 % NaCl, 0,005 % CaCl₂ x 2 H₂O, 5 ml einer 0,01 % Fe(III)citrat-Lösung, 1 ml Spurenelemente-Lösung SL-6 [500 µl/l H₂SO₄, 2,28 g/l MnSO₄ x H₂O, 500 mg/l ZnSO₄ x 7 H₂O, 500 mg H₃BO₃, 25 mg/l CuSO₄ x 5 H₂O 25 mg/l Na₂MoO₄ x 2 H₂O, 45 mg/l CoCl₂ x 6 H₂O]. Am Tag 12 der Kultivierung wurden Zellen mittels Zentrifugation vom Kulturmedium separiert und bei -80°C gelagert.

### B) Amplifikation des für selektive Oxidoreduktase kodierenden Gens

Genomische DNA wurde nach der in "Molecular cloning" von Manniatis & Sambrook beschriebenen Methode extrahiert. Die resultierende Nukleinsäure diente als Matrize für die Polymerase-Kettenreaktion (PCR) mit spezifischen Primern, die von der in der NCBI Datenbank unter der Nummer 76258197 publizierten Gen-Sequenz abgeleitet wurden. Dabei wurden die Primer für eine nachfolgende Klonierung in einen Expressionsvektor 5'-terminal mit Restriktionsschnittstellen für die Endonukleasen Nde I und Hind III bzw. Sph I versehen (SEQ ID NO: 11, SEQ ID NO: 12, SEQ ID NO: 13).

Die Amplifizierung wurde in einem PCR-Puffer [10 mM Tris-HCl, (pH 8,0); 50 mM KCl; 10 mM MgSO₄; 1 mM dNTP Mix; je 20 pMol Primer und 2,5 U Platinum Pfx DNA-Polymerase (Invitrogen)] mit 500 ng genomischer DNA und folgenden Temperatur-Zyklen durchgeführt:

| | |
|---|---|
| Zyklus 1: | 94°C, 2 min |
| Zyklus 2 x 30: | 94°C, 30 sec |
| | 56°C, 30 sec |
| | 68°C, 60 sec |
| Zyklus 3: | 68°C, 7 min |
| | 4°C, ∞ |

Das resultierende PCR-Produkt einer Größe von etwa 750 bp wurde nach der Reinigung über ein 1 % Agarose-Gel mit Hilfe von Endonukleasen *Nde I* und *Hind III,* bzw. mit Endonucleasen *Sph I* und *Hind III* restringiert und in das mit gleichen Endonukleasen behandelte Rückgrad des pET21a Vectors (Novagen) bzw. pQE70 Vectors (Qiagen) ligiert. Nach der Transformation von 2 µl des Ligationsansatzes in *E. coli* Top 10 F' Zellen (Invitrogen) wurden Plasmid-DNAs Ampicillin-(bzw. Kanamycin)-resistenter Kolonien mittels einer Restriktionsanalyse mit den Endonukleasen *Nde I* und *Hind III* bzw. den Endonucleasen *Sph I* und *Hind III* auf das Vorhandensein eines 750 bp großen Inserts getestet. Plasmid-Präparationen aus den für das Fragment positiven Klonen wurden einer Sequenzanalyse unterzogen und anschließend in *Escherichia coli* BL21 Star (Invitrogen), bzw *E. coli* RB791 (genetic stock, Yale) transformiert.

### Beispiel 2 (nicht erfindungsgemäß)

### Expression von rekombinanter Chloroflexus Oxidoreduktase in E.coli

Die mit dem Expressionskonstrukt transformierten *Escherichia coli*-Stämme BL21 Star (Invitrogen, Karlsruhe, Deutschland) bzw. RB791 (E.coli genetic stock, Yale, USA) wurden in 200 ml LB-Medium (1% Tryptone, 0,5% Hefeextrakt, 1% NaCl) mit Ampicillin (50 µg/ml) bzw. Carbenicillin (50 µg/ml) kultiviert, bis eine optische Dichte (OD) von 0,5, gemessen bei 550 nm, erreicht wurde. Die Expression von rekombinantem Protein wurde durch Zugabe von Isopropylthiogalaktosid (IPTG) in einer Konzentration von 0,1 mM induziert. Nach 8 Stunden bzw. 16 Stunden Induktion bei 25°C und 220 Upm wurden die Zellen geerntet und bei -20°C eingefroren. Für den Aktivitätstest wurden 10 mg Zellen mit 500 µl 100 mM TEA Puffer pH 7,0 und 500 µl Glasperlen versetzt und 10 min mittels einer Kugelmühle aufgeschlossen. Das erhaltene Lysat wurde dann verdünnt für die entsprechenden Messungen eingesetzt. Der Aktivitätstest setzte sich wie folgt zusammen: 870 µl 100 mM TEA Puffer pH 7,0, 160 µg NADH, 10 µl verdünntes Zelllysat. Die Reaktion wurde durch Zugabe von 100 µl einer 100 mM Substratlösung zum Reaktionsgemisch gestartet.

Für die Enzymgewinnung in großen Mengen wurden 30 g Zellen in 150 ml Triethanolaminpuffer (100 mM, pH 7, 2 mM MgCl₂, 10 % Gylcerin) resuspendiert und mittels Hochdruckhomogenisator aufgeschlossen. Die Enzymlösung wurde anschließend mit 150 ml Glycerin versetzt und bei -20°C gelagert.

### Beispiel 3

### Anzucht von Organismen und Screening nach reduktiver Umsetzung von Ethylsecodion (Formel III)

Zum Screening wurden die Hefestämme Pichia farinosa DSM 70362, *Candida gropengiesseri* MUCL 29836, *Candida vaccinii* CBS 7318, *Pichia farinosa* DSM 3316, *Saccharomyces cerevisiae* CBS 1508 und *Candida magnoliae* CBS 6396 in folgendem Medium kultiviert: Hefeextrakt (5), Pepton (5) und Glucose (20) (Zahlen in Klammern sind jeweils g/1). Das Medium wurde bei 121 °C sterilisiert und die Hefen wurden ohne weitere pH-Regulierung bei 25°C auf einem Schüttler bei 140 Umdrehungen pro Minute kultiviert.

Die reduktive Umsetzung von Ethylsecodion der Formel III zur entsprechenden Hydroxysecosteroidverbindung wurde in folgenden Ganzzellbiotransformations-Ansätzen getestet:
400 mg frisch geerntete Zellen wurden in einem Ansatz mit 50 mg Glucose, 10 mg Ethylsecodion der Formel III und 900 µl 100 mM Trieethanolamin Puffer (TEA) pH 7,0 Für 24 Stunden bei 28°C und 1400 Upm geschüttelt. Anschließend wurden die Ansätze mit 1 ml Dichlormethan extrahiert, zentrifugiert, mit Stickstoff getrocknet und in Acetonitril aufgenommen zur HPLC-Analyse gegeben.

Die Screeningergebnisse sind in Tabelle 1 zusammengefasst.

**Tabelle 1**

| Stamm Nr. | Mikrorganismus | Umsetzung von Ethylsecodion nach 24 Stunden mit Wt Stämmen |
|---|---|---|
| | | Ansatz 24 h |
| DSM 70362 | Pichia farinosa | 0,7 % |
| MUCL 29836 | Candida gropengiesseri | 0,2 % |
| CBS 7318 | Candida vaccinii | 3,2 % |
| DSM 3316 | Pichia farinosa | 15,8 % |
| CBS 1508 | Saccharomyces cerevisiae | 0,7 % |
| CBS 6396 | Candida magnoliae | 41 % |

Der Stamm CBS 6396 zeigte die höchste Umsetzung von Ethylsecodion und wurde daher als Ausgangsorganismus für die Herstellung einer cDNA Bibliothek ausgesucht.

### Beispiel 4

### Herstellung einer cDNA Bibliothek aus Candida magnoliae CBS 6396 und Clonierung von Oxidoreduktase

### A) Isolierung (gesamt und mRNA) sowie Herstellung der cDNA Bibliothek

600 mg frische Zellen wurden in 2,5 ml eiskaltem LETS-Puffer resuspendiert. Zu dieser Zellsuspension wurden 5 ml (etwa 20 g) in Salpetersäure gewaschener Glasperlen, equilibriert mit 3 ml Phenol (pH 7,0), hinzugegeben. Der gesamte Ansatz wurde dann abwechselnd jeweils 30 sec Vortex, 30 sec Kühlen auf Eis insgesamt 10 min behandelt. Anschließend wurden 5 ml eiskalter LETS-Puffer hinzugegeben und nochmals kräftig mit Vortex gemischt. Diese Zellsuspension wurde für 5 min bei 11000 g bei 4°C zentrifugiert. Die wässrige Phase wurde gewonnen und mit dem gleichen Volumen an Phenol:Chloroform:Isoamylalkohol (24:24:1) zweimal extrahiert. Anschließend folgte die Extraktion mit Chloroform. Nach der letzten Extraktion wurde die Gesamt-RNA durch Zugabe von 1/10 Vol an 5 M LiCl₂ bei -20°C 4 h präzipitiert.
1 mg so gewonnene Gesamt-RNA wurde über Oligo-dT Cellulose (NEB Biolabs) für die Anreicherung der mRNA-Moleküle benutzt. Nach der anschließenden Präzipitation wurden 5 µg mRNA für die cDNA Synthese (pBluescript IIXR cDNA Library Construction kit, Stratagene) verwendet. Die nach den Angaben des Herstellers konstruierte Bibliothek wurde in XL-10 Gold *E.coli* transformiert und auf die Aktivität einer ADH gescreent. Anhand der Extinktionsabnahme mit NADPH bzw. NADH als Cofaktor und Ethylsecodion (Formel III) als Substrat wurde ein Klon (cM4) identifiziert und isoliert. Die Sequenzierung des aus dem Klon isolierten Plasmids mit Primer T7 und Primer T3 resultierte in einer ORF von 789 bp. Dieses Fragment codierte für ein Fusionsprotein einer Größe von 262 Aminosäuren und bestand aus dem a-Fragment der ß-Galactosidase und der Sequenz einer putativen short-chain Alkoholdehydrogenase.

### B) Synthese eines für eine short-chain ADH aus Candida magnoliae CBS 6396 kodierenden Volllänge-Transkripts durch PCR

Es wurden spezifische Primer für eine nachfolgende Klonierung des Volllänge-Transkripts in die passenden Expressionssysteme konstruiert. Dabei wurde 5'-Primer mit einer Erkennungssequenz für *Nde I* bzw*. Sph I* und 3'-Primer mit einer Erkennungssequenz für *Xhol* bzw. *Sacl* modifiziert (SEQ ID NO: 14, SEQ ID NO: 15, SEQ ID NO:16, SEQ ID NO: 17). Plasmid-DNA, isoliert aus dem Klon (cM4) der Expressionsbibliothek von *Candida magnoliae,* diente als Matrize für die Polymerase-Kettenreaktion. Die Amplifizierung wurde in einem PCR-Puffer [10 mM Tris-HCl (pH 8,0); 50 mM KCl; 10 mM MgSO₄; 1 mM dNTP Mix; je 20 pMol Primer und 2,5 U Platinum Pfx DNA-Polymerase (Invitrogen)] mit 50 ng Matrize und folgenden Temperatur-Zyklen durchgeführt:

| | |
|---|---|
| Zyklus 1: | 94°C, 2 min |
| Zyklus 2 x 30: | 94°C, 15 sec |
| | 58°C, 30 sec |
| | 68°C, 75 sec |
| Zyklus 3: | 68°C, 7 min |
| | 4°C, ∞ |

Das resultierende PCR-Produkt wurde nach der Reinigung über ein 1 % Agarose-Gel mit Hilfe der Endonukleasen *Nde I* und *Xho I* bzw. der Endonucleasen *Sph I* und *Sac I* restringiert und in das mit gleichen Endonukleasen behandelte Rückgrad des pET21 a Vectors (Novagen) bzw. pQME70 Vectors ligiert. Nach der Transformation von 2 µl des Ligationsansatzes in *E. coli* Top 10 F' Zellen (Invitrogen) wurden Plasmid-DNAs Ampicillin-(bzw. Kanamycin)-resistenter Kolonien mittels einer Restriktionsanalyse mit den Endonukleasen *Nde I* und *Xhol* bzw. den Endonucleasen *Sph* I und *Sacl* auf das Vorhandensein eines 750 bp großen Inserts getestet. Die Expressionskonstrukte pET21-MgIV und pQME70-MgIV wurden sequenziert. Das für eine short-chain Oxidoreduktase codierende Gen aus *Candida magnoliae* besaß einen offene Leserahmen von insgesamt 729 bp (in SEQ ID NO: 8 enthalten), das einem Protein von 243 Aminosäuren entsprach (SEQ ID NO:3)

### Beispiel 5

### Expression rekombinanter Oxidoreduktase in E.coli Zellen

Kompetente *Escherichia coli* StarBL21(De3)-Zellen (Invitrogen) bzw. RB791-Zellen (E.coli genetic stock, Yale, USA) wurden mit den für die Oxidoreduktase codierenden Expressionskonstrukten pET21-MgIV bzw. pQME70-MgIV transformiert. Die mit dem Expressionskonstrukten transformierten *Escherichia coli*-Kolonien wurden dann in 200 ml LB-Medium (1% Trypton, 0,5% Hefeextrakt, 1% NaCl) mit 50 µg/ml Ampicillin bzw. 40 (µg/ml Kanamycin kultiviert, bis eine optische Dichte von 0,5, gemessen bei 550 nm, erreicht wurde. Die Expression von rekombinantem Protein wurde durch Zugabe von Isopropylthiogalaktosid (IPTG) in einer Konzentration von 0,1 mM induziert. Nach 16 Stunden Induktion bei 25°C und 220 Upm wurden die Zellen geerntet und bei -20°C eingefroren. Für den Aktivitätstest wurden 10 mg Zellen mit 500 µl 100 mM TEA Puffer pH 7,0, 1 mM MgCl₂ und 500 µl Glasperlen versetzt und 10 min mittels einer Kugelmühle aufgeschlossen. Das erhaltene Lysat wurde dann verdünnt für die entsprechenden Messungen eingesetzt.

Der Aktivitätstest setzte sich wie folgt zusammen: 960 µl 100 mM TEA Puffer pH 7,0, IbmM MgCl₂, 160 µg NADPH, 10 µl verdünntes Zelllysat. Die Reaktion wurde durch Zugabe von 10 µl einer 100 mM Substratlösung in 70 % Methanol zum Reaktionsgemisch gestartet.

Für die Enzymgewinnung in großen Mengen wurden 30 g Zellen in 150 ml Triethanolaminpuffer (100 mM, pH 7, 2 mM MgCl₂, 10 % Gylcerin) resuspendiert und mittels Hochdruckhomogenisator aufgeschlossen. Die Enzymlösung wurde anschließend mit 150 ml Glycerin versetzt und bei -20°C gelagert.

### Beispiel 6 (nicht erfindungsgemäß)

### Reduktion von Ethylsecodion (Formel III) mittels Oxidoreduktase SEQ ID NO:1

Für die Reduktion von Ethylsecodion (Formel III) wurde in einem Reaktionsgefäß ein Gemisch aus 800 µl Puffer (100 mM Kaliumphosphat, pH = 7, 2 mM MgCl₂), 1,2 ml 2-Propanol, 0,08 mg NAD, 100 mg Ethylsecodion (Formel III) und 1 ml Enzymsuspension Oxidoreduktase SEQ ID NO:1 (siehe Beispiel 3) für 24 h bei Raumtemperatur unter ständiger Durchmischung inkubiert. Nach 96 h waren >90 % des eingesetzten Ethylsecodions (Formel III) reduziert.

Nach Beendigung der Reaktion wurde das Reaktionsgemisch durch Extraktion mit Dichlormethan aufgearbeitet, die das Produkt enthaltende organische Phase wurde abgetrennt und die 17-beta-Hydroxyverbindung (Ethylsecol) durch Abdampfen/Abdestilieren des Lösungsmittels gewonnen.

Die Umsetzung des Ethylsecodions zum Ethylsecol wurde mittels HPLC verfolgt. Hierzu wurde eine Trennsäule EC125/4 Nucleodur 100-5 C18ec (Machery-Nagel, Düren, Deutschland) mit Acetonitril und Wasser als Laufmittel verwendet. Zur Analytik wurde ein linearer Gradient des Acetonitrilanteils im Laufmittel von 30 % auf 70 % angewendet. Die Identifizierung der Reaktionsprodukte erfolgte durch Vergleich mit Referenzsubstanzen.

### Beispiel 7 (nicht erfindungsgemäß)

### Reduktion von Ethylsecodion (Formel III) mittels Oxidoreduktase SEQ ID NO:2

Für die Reduktion von Ethylsecodion (Formel III) wurde in einem Reaktionsgefäß ein Gemisch aus 250 µl Puffer (100 mM Triethanolamin, pH = 8, 2 mM MgCl₂), 250 µl 4-Methyl-2-pentanol, 0,02 mg NAD, 25 mg Ethylsecodion (Formel III) und 25 µl Enzymsuspension Oxidoreduktase SEQ ID NO:2 (siehe Beispiel 3) für 96 h bei Raumtemperatur unter ständiger Durchmischung inkubiert. Nach 96 h waren >30 % des eingesetzten Ethylsecodions (Formel III) zur Hydroxyverbindung reduziert.

Nach Beendigung der Reaktion wurde das Reaktionsgemisch durch Extraktion mit Dichlormethan aufgearbeitet, die das Produkt enthaltende organische Phase wurde abgetrennt und die 17-beta-Hydroxyverbindung (Ethylsecol) durch Abdampfen/Abdestillieren des Lösungsmittels gewonnen.

### Beispiel 8

### Reduktion von Ethylsecodion (Formel III) mittels Oxidoreduktase SEQ ID NO:3

Für die Reduktion von Ethylsecodion (Formel III) wurde in einem Reaktionsgefäß ein Gemisch aus 100 µl Puffer (100 mM Triethanolamin, pH = 7, 2 mM MgCl₂), 400 µl 4-Methyl-2-pentanol, 0,02 mg NADP, 25 mg Ethylsecodion (Formel III) und 100 µl Enzymsuspension Oxidoreduktase SEQ ID NO:3 (siehe Beispiel 3) für 72 h bei Raumtemperatur unter ständiger Durchmischung inkubiert. Nach 72 h waren >95 % des eingesetzten Ethylsecodions (Formel III) zur Hydroxyverbindung reduziert.

### Beispiel 9 (nicht erfindungsgemäß)

### Reduktion von Ethylsecodion (Formel III) mittels Oxidoreduktase SEQ ID NO:4

Für die Reduktion von Ethylsecodion (Formel III) wurde in einem Reaktionsgefäß ein Gemisch aus 200 µl Puffer (100 mM Triethanolamin, pH = 9, 2 mM MgCl₂), 300 µl 2-Heptanol, 0,025 mg NADP, 100 mg Ethylsecodion (Formel III) und 50 µl Enzymsuspension Oxidoreduktase SEQ ID NO:4 (siehe Beispiel 3) für 72 h bei Raumtemperatur unter ständiger Durchmischung inkubiert. Nach 72 h waren >80 % des eingesetzten Ethylsecodions (Formel III) zur Hydroxyverbindung reduziert.

### Beispiel 10 (nicht erfindungsgemäß)

### Reduktion von Ethylsecodion (Formel III) mittels Oxidoreduktase SEQ ID NO:5

Für die Reduktion von Ethylsecodion (Formel III) wurde in einem Reaktionsgefäß ein Gemisch aus 300 µl Puffer (100 mM Triethanolamin, pH = 7, 2 mM MgCl₂), 1,2 ml 4-Methyl-2-pentanol, 0,12 mg NADP, 150 mg Ethylsecodion (Formel III) und 0,6 ml Enzymsuspension Oxidoreduktase SEQ ID NO:5 (siehe Beispiel 3) für 72 h bei Raumtemperatur unter ständiger Durchmischung inkubiert. Nach 72 h waren >90 % des eingesetzten Ethylsecodions (Formel III) zur Hydroxyverbindung reduziert.

## Patentansprüche

1. Polypeptid mit Oxidoreduktaseaktivität, welches
a) die Aminosäuresequenz SEQ ID NO:3 aufweist oder
b) von der Nukleinsäuresequenz SEQ ID NO:8 kodiert wird und Secodionderivate der allgemeinen Formel I 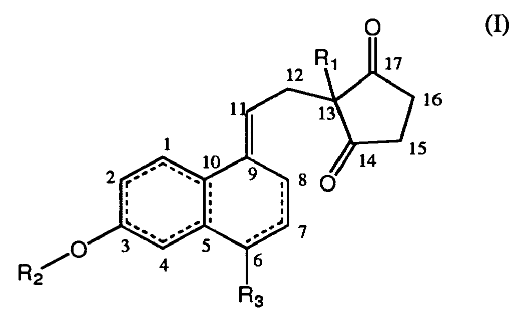
worin die Ringstrukturen kein, ein oder mehrere Heteroatome umfassen,
R₁ Wasserstoff oder eine C₁-C₄-Alkylgruppe ist,
R₂ Wasserstoff, eine C₁-C₈-Alkylgruppe oder eine OH-Schutzgruppe, wie ein Ester, ist,
R₃ Wasserstoff, eine Methylgruppe oder ein Halogenid ist,
das Strukturelement 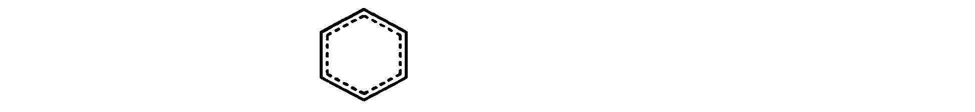 einen Benzolring oder einen C₆-Ring mit 0, 1 oder 2 C-C-Doppelbindungen repräsentiert, an den Positionen 6/7 oder 7/8 gegebenenfalls eine Doppelbindung enthalten ist und der Kohlenstoff an den Positionen 1, 2, 4, 5, 6, 7, 8, 9, 11, 12 und 16 unabhängig mit Wasserstoff, einer C₁-C₄-Alkylgruppe, einem Halogenid oder einer Phenylgruppe substituiert ist, in Gegenwart von NADH oder NADPH als Cofactor reduziert.

2. Polypeptid mit Oxidoreduktaseaktivität, welches Secodionderivate der allgemeinen Formel I 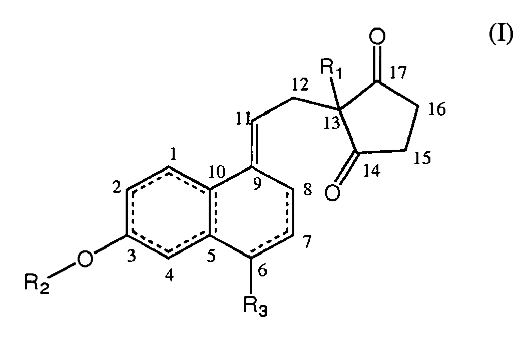 worin die Ringstrukturen kein, ein oder mehrere Heteroatome umfassen,
R₁ Wasserstoff oder eine C₁-C₄-Alkylgruppe ist,
R₂ Wasserstoff, eine C₁-C₈-Alkylgruppe oder OH-Schutzgruppe, wie ein Ester, ist,
R₃ Wasserstoff, eine Methylgruppe oder ein Halogenid ist,
das Strukturelement 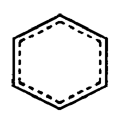 einen Benzolring oder einen C₆-Ring mit 0, 1 oder 2 C-C-Doppelbindungen repräsentiert, an den Positionen 6/7 oder 7/8 gegebenenfalls eine Doppelbindung enthalten ist und der Kohlenstoff an den Positionen 1, 2, 4, 5, 6, 7, 8, 9, 11, 12 und 16 unabhängig mit Wasserstoff, einer C₁-C₄-Alkylgruppe, einem Halogenid oder einer Phenylgruppe substituiert ist,
in Gegenwart von NADH oder NADPH als Cofactor reduziert und von einer Nukleinsäuresequenz kodiert wird, die mit der Nukleinsäuresequenz SEQ ID NO:8 zumindest 80% Identität aufweist und unter stringenten Bedingungen hybridisiert, wobei die stringenten Bedingungen die Hybridisierung in 0.7-1 M NaCl-Lösung bei 60°C umfassen.

3. Isoliertes Polypeptid nach Anspruch 1 oder 2, erhältlich aus Candida magnoliae CBS 6396.

## Claims

1. Polypeptide having oxidoreductase activity which
a) has the amino acid sequence SEQ ID NO: 3 or
b) is encoded by the nucleic acid sequence SEQ ID NO: 8 and reduces secodione derivatives of the general formula I 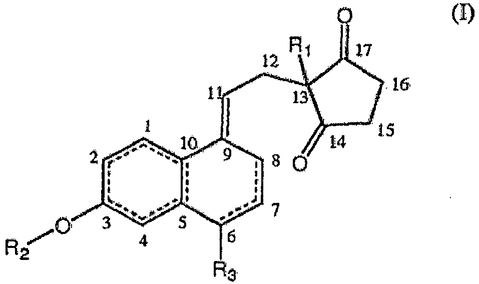
where the ring structures comprise no, one or a plurality of heteroatoms,
R₁ is hydrogen or a C₁-C₄ alkyl group,
R₂ is hydrogen, a C₁-C₈ alkyl group or an OH- protecting group, such as an ester,
R₃ is hydrogen, a methyl group or a halide,
the structural element 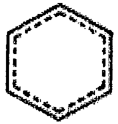 represents a benzene ring or a C₆ ring having 0, 1 or 2 C-C double bonds, optionally at the positions 6/7 or 7/8 a double bond is present and the carbon at the positions 1, 2, 4, 5, 6, 7, 8, 9, 11, 12 and 16 is independently substituted with hydrogen, a C₁-C₄ alkyl group, a halide or a phenyl group, in the presence of NADH or NADPH as cofactor.

2. Polypeptide having oxidoreductase activity which reduces secodione derivatives of the general formula I 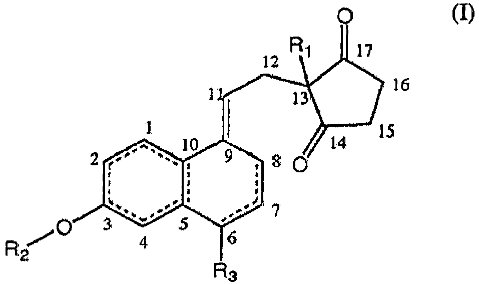 where the ring structures comprise no, one or a plurality of heteroatoms,
R₁ is hydrogen or a C₁-C₄ alkyl group,
R₂ is hydrogen, a C₁-C₈ alkyl group or OH- protecting group, such as an ester,
R₃ is hydrogen, a methyl group or a halide,
the structural element 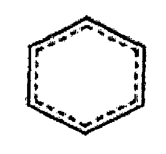 represents a benzene ring or a C₆ ring having 0, 1 or 2 C-C double bonds, optionally at the positions 6/7 or 7/8 a double bond is present and the carbon at the positions 1, 2, 4, 5, 6, 7, 8, 9, 11, 12 and 16 is independently substituted with hydrogen, a C₁-C₄ alkyl group, a halide or a phenyl group, in the presence of NADH or NADPH as cofactor and is encoded by a nucleic acid sequence which has at least 80 % identity with the nucleic acid sequence SEQ ID NO: 8 and hybridizes under stringent conditions, wherein the stringent conditions comprise hybridization in 0.7-1 M NaCl solution at 60 °C.

3. Isolated polypeptide according to Claim 1 or 2, obtainable from Candida magnoliae CBS 6396.

## Revendications

1. Polypeptide avec une activité d'oxydoréductase, qui :
a) présente la séquence d'acides aminés SEQ ID NO :3 ou
b) est codé par la séquence d'acide nucléique SEQ ID NO :8 et réduit des dérivés de sécodione de formule générale I : 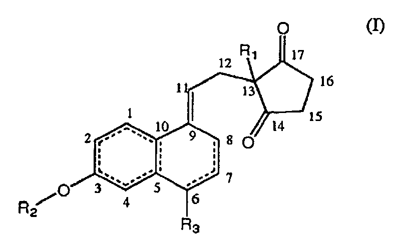
où les structures cycliques ne comprennent pas d'hétéroatomes ou en présentent un ou plusieurs,
R₁ est de l'hydrogène ou un groupe alkyle en C₁-C₄,
R₂ est de l'hydrogène, un groupement alkyle en C₁-C₈ ou un groupement de protection de OH, tel qu'un ester,
R₃ est de l'hydrogène, un groupement méthyle ou un halogénure, l'élément strûcturel : 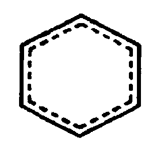 représente un cycle de benzène ou un cycle en C₆ avec 0, 1 ou 2 doubles liaisons C-C, une double liaison est contenue éventuellement dans les positions 6/7 ou 7/8 et le carbone est substitué dans les positions 1, 2, 4, 5, 6, 7, 8, 9, 11, 12 et 16 indépendamment par de l'hydrogène, un groupement alkyle en C₁-C₄, un halogénure ou un groupement phényle, en présence de NADH ou de NADPH comme cofacteurs.

2. Polypeptide avec une activité d'oxydoréductase, qui réduit des dérivés de sécodione de formule générale I : 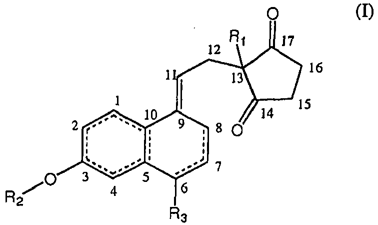 où les structures cycliques ne comprennent pas d'hétéroatomes ou en comprennent un ou plusieurs,
R₁ est de l'hydrogène ou un groupement alkyle en C₁-C₄,
R₂ est de l'hydrogène, un groupement alkyle en C₁-C₈ ou un groupement de protection de OH, tel qu'un ester,
R₃ est de l'hydrogène, un groupement méthyle ou un halogénure, l'élément structurel : 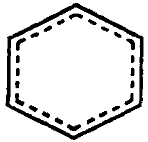 représente un cycle de benzène ou un cycle en C₆ avec 0, 1 ou 2 doubles liaisons C-C, une double liaison est éventuellement présente dans les positions 6/7 ou 7/8 et le carbone est substitué dans les positions 1, 2, 4, 5, 6, 7, 8, 9, 11, 12 et 16 indépendamment par de l'hydrogène, un groupement alkyle en C₁-C₄, un halogénure ou un groupement phényle, en présence de NADH ou de NADPH comme cofacteurs et est codé par une séquence d'acide nucléique, qui présente une identité d'au moins 80% avec la séquence d'acide nucléique SEQ ID NO :8 et est hybridée dans des conditions stringentes, dans lequel les conditions stringentes comprennent l'hybridation dans 0,7 à 1 M de solution de NaCl à 60°C.

3. Polypeptide isolé selon la revendication 1 ou la revendication 2, qui peut être tiré de la souche Candida magnoliae CBS 6396.


### SEQUENCE LISTING

<110> IEP GmbH
<120> Verfahren zur enantioselektiven enzymatischen Reduktion von Secodionderivaten
<130> I 12274A
<140> EP 11177932.8
   <141> 2007-12-07
<160> 42
<170> PatentIn version 3.3
<210> 1
   <211> 252
   <212> PRT
   <213> Chloroflexus auratiacus
<400> 1
   Met Glu Pro Pro Phe Ile Gly Lys Val Ala Leu Val Thr Gly Ala Ala
   1               5                   10                  15
   Ala Gly Ile Gly Arg Ala Ser Ala Leu Ala Phe Ala Arg Glu Gly Ala
               20                  25                  30
   Lys Val Val Val Ala Asp Val Asn Val Glu Gly Gly Glu Glu Thr Ile
           35                  40                  45
   Ala Leu Cys Arg Ala Leu Asn Thr Asp Ala Met Phe Val Arg Cys Asp
       50                  55                  60
   Val Ser Gln Arg Asp Glu Val Glu Arg Leu Ile Ala Leu Ala Val Asp
   65                  70                  75                  80
   Thr Phe Gly Arg Ile Asp Phe Ala His Asn Asn Ala Gly Ile Glu Gly
                   85                  90                  95
   Val Gln Ala Met Leu Ala Asp Tyr Pro Glu Glu Val Trp Asp Arg Val
               100                 105                 110
   Ile Glu Ile Asn Leu Lys Gly Val Trp Leu Cys Met Lys Tyr Glu Ile
           115                 120                 125
   Arg His Met Leu Lys Gln Gly Gly Gly Ala Ile Val Asn Thr Ser Ser
       130                 135                 140
   Val Ala Gly Leu Ala Gly Ser Arg Gly Val Ser Ala Tyr Val Ala Ser
   145                 150                 155                 160
   Lys His Gly Ile Val Gly Ile Thr Lys Ala Ala Ala Leu Glu Tyr Ala
                   165                 170                 175
   Arg Asn Gly Ile Arg Val Asn Ala Ile Cys Pro Gly Thr Ile His Thr
               180                 185                 190
   Ala Met Ile Asp Arg Phe Thr Gln Gly Asp Pro Gln Leu Leu Ala Gln
           195                 200                 205
   Phe Ala Glu Gly Glu Pro Ile Gly Arg Leu Gly Ser Pro Glu Glu Val
       210                 215                 220
   Ala Asn Ala Val Ile Trp Leu Cys Ser Asp Lys Ala Ser Phe Val Thr
   225                 230                 235                 240
   Gly Ala Thr Leu Ala Val Asp Gly Gly Arg Leu Ala
                   245                 250
<210> 2
   <211> 249
   <212> PRT
   <213> Rubrobacter xylanophilus
<400> 2
   Met Leu Glu Gly Lys Val Ala Val Ile Thr Gly Ala Gly Ser Gly Ile
   1               5                   10                  15
   Gly Arg Ala Thr Ala Leu Lys Phe Ala Arg Glu Gly Ala Arg Val Val
               20                  25                  30
   Ala Ala Glu Leu Asp Glu Arg Gly Gly Glu Gly Val Val Arg Glu Val
           35                  40                  45
   Arg Ser Leu Gly Gly Glu Ala Val Phe Val Arg Thr Asp Val Ser Glu
       50                  55                  60
   Phe Ala Gln Val Glu Asp Ala Val Glu Arg Ala Val Gly Glu Tyr Gly
   65                  70                  75                  80
   Thr Leu Asp Val Met Phe Asn Asn Ala Gly Ile Gly His Tyr Ala Pro
                   85                  90                  95
   Leu Leu Glu His Glu Pro Glu His Tyr Asp Arg Val Val Arg Val Asn
               100                 105                 110
   Gln Tyr Gly Val Tyr Tyr Gly Ile Leu Ala Ala Gly Arg Lys Met Val
           115                 120                 125
   Ala Leu Lys Asn Pro Gly Leu Ile Ile Asn Thr Ala Ser Val Tyr Ala
       130                 135                 140
   Phe Leu Ala Ser Pro Gly Val Ile Gly Tyr His Ala Ala Lys Gly Ala
   145                 150                 155                 160
   Val Lys Met Met Thr Gln Ala Ala Ala Leu Glu Leu Ala Pro His Gly
                   165                 170                 175
   Ile Arg Val Val Ala Ile Ala Pro Gly Gly Val Asp Thr Pro Ile Ile
               180                 185                 190
   Gln Gly Tyr Lys Asp Met Gly Leu Gly Glu Arg Leu Ala Arg Gly Gln
           195                 200                 205
   Met Arg Arg Arg Leu Gln Thr Pro Glu Gln Ile Ala Gly Ala Val Ala
       210                 215                 220
   Leu Leu Ala Thr Asp Glu Ala Asp Ala Ile Asn Gly Ser Val Val Met
   225                 230                 235                 240
   Thr Asp Asp Gly Tyr Ala Glu Phe Lys
                   245
<210> 3
   <211> 243
   <212> PRT
   <213> Candida magnoliae
<400> 3
   Met Ser Ala Thr Ser Asn Ala Leu Ile Thr Gly Ala Ser Arg Gly Met
   1               5                   10                  15
   Gly Glu Ala Thr Ala Ile Lys Leu Ala Leu Glu Gly Tyr Ser Val Thr
               20                  25                  30
   Leu Ala Ser Arg Gly Ile Glu Gln Leu Asn Ala Ile Lys Glu Lys Leu
           35                  40                  45
   Pro Ile Val Lys Lys Gly Gln Gln His Tyr Val Trp Gln Leu Asp Leu
       50                  55                  60
   Ser Asp Ile Glu Ala Ala Ser Thr Phe Lys Gly Ala Pro Leu Pro Ala
   65                  70                  75                  80
   Ser Ser Tyr Asp Val Phe Phe Ser Asn Ala Gly Val Val Asp Phe Ala
                   85                  90                  95
   Pro Phe Ala Asp Gln Ser Glu Thr Ala Gln Lys Asp Leu Phe Thr Val
               100                 105                 110
   Asn Leu Leu Ser Pro Val Ala Leu Thr Lys Thr Ile Val Lys Ala Ile
           115                 120                 125
   Ala Asp Lys Pro Arg Glu Thr Pro Ala His Ile Ile Phe Thr Ser Ser
       130                 135                 140
   Ile Val Gly Ile Arg Gly Val Pro Asn Val Ala Val Tyr Ser Ala Thr
   145                 150                 155                 160
   Lys Gly Ala Ile Asp Ser Phe Ala Arg Ser Leu Ala Arg Glu Phe Gly
                   165                 170                 175
   Pro Lys Asn Ile His Val Asn Cys Val Asn Pro Gly Thr Thr Arg Thr
               180                 185                 190
   Glu Met Thr Lys Gly Val Asp Leu Ala Ala Phe Gly Asp Val Pro Ile
           195                 200                 205
   Lys Gly Trp Ile Glu Val Asp Ala Ile Ala Asp Ala Val Leu Phe Leu
       210                 215                 220
   Ile Lys Ser Lys Asn Ile Thr Gly Gln Ser Leu Val Val Asp Asn Gly
   225                 230                 235                 240
   Phe Gly Val
<210> 4
   <211> 241
   <212> PRT
   <213> Candida magnoliae
<400> 4
   Met Thr Ser Thr Pro Asn Ala Leu Ile Thr Gly Gly Ser Arg Gly Ile
   1               5                   10                  15
   Gly Ala Ser Ala Ala Ile Lys Leu Ala Gln Glu Gly Tyr Ser Val Thr
               20                  25                  30
   Leu Ala Ser Arg Asp Leu Glu Lys Leu Thr Glu Val Lys Asp Lys Leu
           35                  40                  45
   Pro Ile Val Arg Gly Gly Gln Lys His Tyr Val Trp Gln Leu Asp Leu
       50                  55                  60
   Ala Asp Val Glu Ala Ala Ser Ser Phe Lys Ala Ala Pro Leu Pro Ala
   65                  70                  75                  80
   Ser Ser Tyr Asp Leu Phe Val Ser Asn Ala Gly Ile Ala Gln Phe Ser
                   85                  90                  95
   Pro Thr Ala Glu His Thr Asn Ser Glu Trp Leu Asn Ile Met Thr Ile
               100                 105                 110
   Asn Leu Val Ser Pro Ile Ala Leu Thr Lys Ala Leu Leu Gln Ala Val
           115                 120                 125
   Ser Gly Arg Ser Ser Glu Asn Pro Phe Gln Ile Val Phe Ile Ser Ser
       130                 135                 140
   Val Ala Ala Leu Arg Gly Val Ala Gln Thr Ala Val Tyr Ser Ala Ser
   145                 150                 155                 160
   Lys Ala Gly Thr Asp Gly Phe Ala Arg Ser Leu Ala Arg Glu Leu Gly
                   165                 170                 175
   Pro Gln Gly Val His Val Asn Val Val Asn Pro Gly Trp Thr Lys Thr
               180                 185                 190
   Asp Met Thr Glu Gly Val Glu Thr Pro Lys Asp Met Pro Ile Lys Gly
           195                 200                 205
   Trp Ile Gln Pro Glu Ala Ile Ala Asp Ala Val Val Phe Leu Ala Arg
       210                 215                 220
   Ser Lys Asn Ile Thr Gly Ala Asn Ile Val Val Asp Asn Gly Phe Ser
   225                 230                 235                 240
   Thr
<210> 5
   <211> 241
   <212> PRT
   <213> Candida magnoliae
<400> 5
   Met Thr Thr Thr Ser Asn Ala Leu Val Thr Gly Gly Ser Arg Gly Ile
   1               5                   10                  15
   Gly Ala Ala Ser Ala Ile Lys Leu Ala Gln Glu Gly Tyr Asn Val Thr
               20                  25                  30
   Leu Ala Ser Arg Ser Val Asp Lys Leu Asn Glu Val Lys Ala Lys Leu
           35                  40                  45
   Pro Ile Val Gln Asp Gly Gln Lys His Tyr Ile Trp Glu Leu Asp Leu
       50                  55                  60
   Ala Asp Val Glu Ala Ala Ser Ser Phe Lys Gly Ala Pro Leu Pro Ala
   65                  70                  75                  80
   Arg Ser Tyr Asp Val Phe Val Ser Asn Ala Gly Val Ala Ala Phe Ser
                   85                  90                  95
   Pro Thr Ala Asp His Asp Asp Lys Glu Trp Gln Asn Leu Leu Ala Val
               100                 105                 110
   Asn Leu Ser Ser Pro Ile Ala Leu Thr Lys Ala Leu Leu Lys Asp Val
           115                 120                 125
   Ser Glu Arg Pro Val Asp Lys Pro Leu Gln Ile Ile Tyr Ile Ser Ser
       130                 135                 140
   Val Ala Gly Leu His Gly Ala Ala Gln Val Ala Val Tyr Ser Ala Ser
   145                 150                 155                 160
   Lys Ala Gly Leu Asp Gly Phe Met Arg Ser Val Ala Arg Glu Val Gly
                   165                 170                 175
   Pro Lys Gly Ile His Val Asn Ser Ile Asn Pro Gly Tyr Thr Lys Thr
               180                 185                 190
   Glu Met Thr Ala Gly Ile Glu Ala Leu Pro Asp Leu Pro Ile Lys Gly
           195                 200                 205
   Trp Ile Glu Pro Glu Ala Ile Ala Asp Ala Val Leu Phe Leu Ala Lys
       210                 215                 220
   Ser Lys Asn Ile Thr Gly Thr Asn Ile Val Val Asp Asn Gly Leu Ile
   225                 230                 235                 240
   Ala
<210> 6
   <211> 759
   <212> DNA
   <213> Chloroflexus aurantiacus
<400> 6
   atggagccac ctttcattgg gaaggttgcg ctggtcaccg gcgcagcagc cggtattggt      60
   cgtgcttcag cactggcgtt tgcccgtgag ggtgccaagg ttgtcgttgc tgatgtgaat     120
   gtcgagggcg gggaagagac gattgcgctg tgtcgggctt tgaataccga tgcaatgttc     180
   gtgcgttgtg atgtttcgca acgcgatgaa gtggagcgat taattgctct ggcagttgac     240
   acgttcggtc ggatcgactt tgcgcacaac aacgccggga ttgaaggcgt gcaggcaatg     300
   ctggccgatt atcccgaaga ggtctgggat cgggtgatcg agatcaacct caaaggggtc     360
   tggttgtgta tgaagtacga aatccggcac atgctcaagc agggtggcgg tgcgattgtg     420
   aatacctcat cggtcgccgg tctggccgga tcacgtggcg tttcggcgta tgtagccagc     480
   aagcacggta ttgttggtat taccaaagcg gcagcccttg agtatgcgcg taacggtatt     540
   cgtgtcaacg caatctgtcc aggtacgatt catactgcga tgatcgaccg ctttacccag     600
   ggtgatcccc aactgcttgc ccagttcgct gagggtgaac cgattggtcg gctcggctcg     660
   cctgaagagg tcgccaatgc ggtgatctgg ctctgctcag ataaggcttc gtttgtgacc     720
   ggagcgacac tggcggttga tggtggccgc ctggcgtaa                            759
<210> 7
   <211> 750
   <212> DNA
   <213> Rubrobacter xylanophilus
<400> 7
   atgctcgagg ggaaggtcgc ggtcatcacg ggggccggaa gcggcatagg ccgggccacc      60
   gcgctcaagt tcgcccgcga gggggcccgg gtcgtcgccg ccgagctcga cgagcgcggc     120
   ggggaggggg tggtccggga ggtgcgcagc ctcgggggcg aggcggtctt cgtccggacc     180
   gacgtctcgg agttcgcgca ggtggaggac gccgtcgagc gggcggtcgg ggagtacggc     240
   accctcgacg tgatgttcaa caacgccggc atcgggcact acgcccccct gctggagcac      300
   gagcccgagc actacgaccg ggtggtccgg gtgaaccagt acggcgtcta ctacgggata     360
   ctcgccgccg ggagaaagat ggtcgccctg aagaaccccg gcttgatcat caacaccgcc     420
   tcggtctacg ccttcctcgc ctcgccgggg gtcatcggct accacgccgc caagggggcg     480
   gtcaagatga tgacccaggc ggcggcgctg gagctcgccc cgcacggcat aagggtcgtc     540
   gccatcgccc cgggcggggt ggacaccccc atcatccagg gctacaagga catggggctc     600
   ggcgagaggc tggcccgcgg ccagatgcgc cgccggctcc agacccccga gcagatcgcc     660
   ggggcggtcg ccctgctcgc caccgacgag gccgacgcca taaacggctc ggtggtcatg     720
   accgacgacg gctacgcgga gttcaagtag                                      750
<210> 8
   <211> 732
   <212> DNA
   <213> Canadida magnoliae
<400> 8
   atgtctgcta cttcgaacgc tcttatcact ggtgccagcc gcggaatggg cgaggccaca      60
   gctattaagc ttgcccttga ggggtacagc gtcacccttg catcacgcgg tattgagcag     120
   ctcaatgcca tcaaggaaaa actacccatc gtgaagaagg gccagcagca ctacgtttgg     180
   cagctcgatc ttagtgacat cgaggcggct tccaccttca agggggctcc tctgcctgcc     240
   agcagctacg acgtgttctt cagcaacgcc ggtgtggtgg actttgctcc gttcgcagac     300
   caaagcgaga ctgcgcaaaa ggacctgttc acggttaacc tgctgtcgcc tgttgcgttg     360
   accaagacca ttgttaaggc catcgccgac aagccccgcg agacgcctgc tcacattatc     420
   ttcacctcgt ccattgtcgg aattcgcggt gttcccaacg tggcggtcta cagcgccacc     480
   aagggcgcga ttgacagctt tgcgcgctcg cttgctcgtg agttcggtcc caagaacatc     540
   cacgttaact gcgtgaaccc gggcacgacg cgcaccgaga tgacaaaggg cgttgatctc     600
   gcggctttcg gcgatgttcc tatcaagggc tggatcgagg tcgatgcgat tgccgacgct     660
   gtgctgtttt tgatcaagtc caagaacatc actggccagt cgctcgttgt tgacaacgga     720
   ttcggtgttt aa                                                         732
<210> 9
   <211> 726
   <212> DNA
   <213> Candida magnoliae
<400> 9
   atgacatcta cacctaatgc cctcatcacg ggaggcagcc gcggcattgg cgcttccgcc      60
   gccatcaaac tggctcaaga agggtacagc gtcacgctgg cgtcccgcga ccttgagaaa     120
   cttactgagg tcaaggacaa gctgccaatc gtgagaggtg gacagaaaca ctacgtttgg     180
   cagctcgatc ttgccgatgt ggaggctgca tcgtctttca aggcggctcc tctgccggcc     240
   agcagctacg atttgtttgt ttcgaacgcc ggaattgccc agttctcgcc tacggcagag     300
   catactaata gtgagtggct gaacattatg accattaact tagtgtcccc gattgccctg     360
   acgaaggctc ttttgcaggc cgtttctggg aggtcgagcg agaacccgtt tcagatcgtc     420
   ttcatctcgt cggttgcagc actacgtggc gttgcacaaa cggccgtcta cagtgcgtcg     480
   aaggctggta ctgatggatt cgcacgctca cttgctcgcg aactaggtcc tcaaggtgtt     540
   catgtgaacg tggtgaaccc tggctggact aagacagaca tgacggaagg agtcgaaacc     600
   ccaaaggaca tgcccattaa gggctggatc cagcctgagg caattgctga tgctgtagta     660
   ttccttgcga ggtcgaaaaa cattaccggc gcgaatattg tagtggacaa tggtttctcg     720
   acgtaa                                                                726
<210> 10
   <211> 726
   <212> DNA
   <213> Candida magnoliae
<400> 10
   atgacgacta cttcaaacgc gcttgtcact ggaggcagcc gcggcattgg cgctgcctcc      60
   gccattaagc tggctcagga gggctacaat gttacgctgg cctctcgcag tgttgataaa     120
   ctgaatgaag taaaggcgaa actcccaatt gtacaggacg ggcagaagca ctacatttgg     180
   gaactcgatc tggctgatgt ggaagctgct tcgtcgttca agggtgctcc tttgcctgct     240
   cgcagctacg acgtctttgt ttcgaacgcg ggcgtcgctg cgttctcgcc cacagccgac     300
   cacgatgata aggagtggca gaacttgctt gccgtgaact tgtcgtcgcc cattgccctc     360
   acgaaggccc tcttgaagga tgtctccgaa aggcctgtgg acaagccact gcagattatc    420
   tacatttcgt cggtggccgg cttgcatggc gccgcgcagg tcgccgtgta cagtgcatct    480
   aaggccggtc ttgatggttt tatgcgctcc gtcgcccgtg aggtgggccc gaagggcatc    540
   catgtgaact ccatcaaccc cggatacacg aagactgaaa tgaccgcggg cattgaagcc    600
   cttcctgatt tgcctatcaa ggggtggatc gagcccgagg caattgctga cgcggttctg    660
   tttctggcaa agtccaagaa tatcaccggc acaaacattg tggtcgacaa tggcttgatt    720
   gcttaa                                                                726
<210> 11
   <211> 38
   <212> DNA
   <213> künstliche Sequenz
<220>
   <221> misc_feature
   <222> (1)..(38)
   <223> Primer für Klonierung von Oxidoreduktasegen SEQ ID NO: 6 aus Chloroflexus aurantiacus in Expressionsvektor
<400> 11
   ggaattccat atgatggagc cacctttcat tgggaagg 38
<210> 12
   <211> 34
   <212> DNA
   <213> künstliche Sequenz
<220>
   <221> misc_feature
   <222> (1)..(34)
   <223> Primer für Klonierung von Oxidoreduktasegen SEQ ID NO: 6 aus Chloroflexus aurantiacus in Expressionsvektor
<400> 12
   cccaagctta ttattacgcc aggcggccac catc 34
<210> 13
   <211> 38
   <212> DNA
   <213> künstliche Sequenz
<220>
   <221> misc_feature
   <222> (1)..(38)
   <223> Primer für Klonierung von Oxidoreduktasegen SEQ ID NO: 6 aus Chloroflexus aurantiacus in Expressionsvektor
<400> 13
   cacatgcatg cagatggagc cacctttcat tgggaagg 38
<210> 14
   <211> 35
   <212> DNA
   <213> künstliche Sequenz
<220>
   <221> misc_feature
   <222> (1)..(35)
   <223> Primer für Klonierung von Oxidoreduktasegen SEQ ID NO: 8 aus Candida magnoliae in Expressionsvektor
<400> 14
   ggaattccat atgatgtctg ctacttcgaa cgctc 35
<210> 15
   <211> 33
   <212> DNA
   <213> künstliche Sequenz
<220>
   <221> misc_feature
   <222> (1)..(33)
   <223> Primer für Klonierung von Oxidoreduktasegen SEQ ID NO: 8 aus Candida magnoliae in Expressionsvektor
<400> 15
   ccgctcgagt tattaaacac cgaatccgtt gtc 33
<210> 16
   <211> 35
   <212> DNA
   <213> künstliche Sequenz
<220>
   <221> misc_feature
   <222> (1)..(35)
   <223> Primer für Klonierung von Oxidoreduktasegen SEQ ID NO: 8 aus Candida magnoliae in Expressionsvektor
<400> 16
   cacatgcatg cagatgtctg ctacttcgaa cgctc 35
<210> 17
   <211> 34
   <212> DNA
   <213> künstliche Sequenz
<220>
   <221> misc_feature
   <222> (1)..(34)
   <223> Primer für Klonierung von Oxidoreduktasegen SEQ ID NO: 8 aus Candida magnoliae in Expressionsvektor
<400> 17
   gcccgagctc ttattaaaca ccgaatccgt tgtc 34
<210> 18
   <211> 12
   <212> PRT
   <213> künstliche Sequenz
<220>
   <221> PEPTIDE
   <222> (1)..(12)
   <223> Oxidoreduktase-Partialsequenz
<400> 18
   Asn Ala Leu Val Thr Gly Ala Ser Arg Gly Ile Gly
   1               5                   10
<210> 19
   <211> 12
   <212> PRT
   <213> künstliche Sequenz
<220>
   <221> PEPTIDE
   <222> (1) . . (12)
   <223> Oxidoreduktase-Partialsequenz
<400> 19
   Asn Ala Leu Val Thr Gly Gly Ser Arg Gly Ile Gly
   1               5                   10
<210> 20
   <211> 12
   <212> PRT
   <213> künstliche Sequenz
<220>
   <221> PEPTIDE
   <222> (1)..(12)
   <223> Oxidoreduktase-Partialsequenz
<400> 20
   Asn Ala Leu Ile Thr Gly Gly Ser Arg Gly Ile Gly
   1               5                   10
<210> 21
   <211> 12
   <212> PRT
   <213> künstliche Sequenz
<220>
   <221> PEPTIDE
   <222> (1)..(12)
   <223> Oxidoreduktase-Partialsequenz
<400> 21
   Asn Ala Leu Ile Thr Gly Ala Ser Arg Gly Ile Gly
   1               5                   10
<210> 22
   <211> 12
   <212> PRT
   <213> künstliche Sequenz
<220>
   <221> PEPTIDE
   <222> (1)..(12)
   <223> Oxidoreduktase-Partialsequenz
<400> 22
   Asn Ala Leu Ile Thr Gly Gly Ser Arg Gly Met Gly
   1               5                   10
<210> 23
   <211> 12
   <212> PRT
   <213> künstliche Sequenz
<220>
   <221> PEPTIDE
   <222> (1)..(12)
   <223> Oxidoreduktase-Partialsequenz
<400> 23
   His Ala Leu Val Thr Gly Ala Ser Arg Gly Ile Gly
   1               5                   10
<210> 24
   <211> 7
   <212> PRT
   <213> künstliche Sequenz
<220>
   <221> PEPTIDE
   <222> (1)..(7)
   <223> Oxidoreduktase-Partialsequenz
<400> 24
   Gly Tyr Ser Val Thr Leu Ala
   1               5
<210> 25
   <211> 7
   <212> PRT
   <213> künstliche Sequenz
<220>
   <221> PEPTIDE
   <222> (1)..(7)
   <223> Oxidoreduktase-Partialsequenz
<400> 25
   Gly Tyr Asn Val Thr Leu Ala
   1               5
<210> 26
   <211> 7
   <212> PRT
   <213> künstliche Sequenz
<220>
   <221> PEPTIDE
   <222> (1)..(7)
   <223> Oxidoreduktase-Partialsequenz
<400> 26
   Gly Tyr Ser Val Thr Leu Val
   1               5
<210> 27
   <211> 7
   <212> PRT
   <213> künstliche Sequenz
<220>
   <221> PEPTIDE
   <222> (1)..(7)
   <223> Oxidoreduktase-Partialsequenz
<400> 27
   Gly Tyr Asn Val Thr Leu Val
   1               5
<210> 28
   <211> 8
   <212> PRT
   <213> künstliche Sequenz
<220>
   <221> PEPTIDE
   <222> (1)..(8)
   <223> Oxidoreduktase-Partialsequenz
<400> 28
   Phe Lys Gly Ala Pro Leu Pro Ala
   1               5
<210> 29
   <211> 8
   <212> PRT
   <213> künstliche Sequenz
<220>
   <221> PEPTIDE
   <222> (1)..(8)
   <223> Oxidoreduktase-Partialsequenz
<400> 29
   Phe Lys Ala Ala Pro Leu Pro Ala
   1               5
<210> 30
   <211> 6
   <212> PRT
   <213> künstliche Sequenz
<220>
   <221> PEPTIDE
   <222> (1)..(6)
   <223> Oxidoreduktase-Partialsequenz
<400> 30
   Phe Val Ser Asn Ala Gly
   1               5
<210> 31
   <211> 6
   <212> PRT
   <213> künstliche Sequenz
<220>
   <221> PEPTIDE
   <222> (1)..(6)
   <223> Oxidoreduktase-Partialsequenz
<400> 31
   Phe Phe Ser Asn Ala Gly
   1               5
<210> 32
   <211> 6
   <212> PRT
   <213> künstliche Sequenz
<220>
   <221> PEPTIDE
   <222> (1)..(6)
   <223> Oxidoreduktase-Partialsequenz
<400> 32
   Phe Val Cys Asn Ala Gly
   1               5
<210> 33
   <211> 6
   <212> PRT
   <213> künstliche Sequenz
<220>
   <221> PEPTIDE
   <222> (1)..(6)
   <223> Oxidoreduktase-Partialsequenz
<400> 33
   Phe Val Ala Asn Ala Gly
   1               5
<210> 34
   <211> 9
   <212> PRT
   <213> künstliche Sequenz
<220>
   <221> PEPTIDE
   <222> (1)..(9)
   <223> Oxidoreduktase-Partialsequenz
<400> 34
   Ser Pro Ile Ala Leu Thr Lys Ala Leu
   1               5
<210> 35
   <211> 9
   <212> PRT
   <213> künstliche Sequenz
<220>
   <221> PEPTIDE
   <222> (1)..(9)
   <223> Oxidoreduktase-Partialsequenz
<400> 35
   Ser Pro Val Ala Leu Thr Lys Thr Ile
   1               5
<210> 36
   <211> 9
   <212> PRT
   <213> künstliche Sequenz
<220>
   <221> PEPTIDE
   <222> (1)..(9)
   <223> Oxidoreduktase-Partialsequenz
<400> 36
   Ser Pro Ile Ala Leu Thr Lys Thr Leu
   1               5
<210> 37
   <211> 9
   <212> PRT
   <213> künstliche Sequenz
<220>
   <221> PEPTIDE
   <222> (1)..(9)
   <223> Oxidoreduktase-Partialsequenz
<400> 37
   Ser Pro Val Ala Met Thr Lys Ala Leu
   1               5
<210> 38
   <211> 9
   <212> PRT
   <213> künstliche Sequenz
<220>
   <221> PEPTIDE
   <222> (1)..(9)
   <223> Oxidoreduktase-Partialsequenz
<400> 38
   Ser Gln Ile Ala Leu Thr Lys Ala Leu
   1               5
<210> 39
   <211> 7
   <212> PRT
   <213> künstliche Sequenz
<220>
   <221> PEPTIDE
   <222> (1)..(7)
   <223> Oxidoreduktase-Partialsequenz
<400> 39
   Ala Val Tyr Ser Ala Ser Lys
   1               5
<210> 40
   <211> 7
   <212> PRT
   <213> künstliche Sequenz
<220>
   <221> PEPTIDE
   <222> (1)..(7)
   <223> Oxidoreduktase-Partialsequenz
<400> 40
   Ala Val Tyr Ser Ala Thr Lys
   1               5
<210> 41
   <211> 6
   <212> PRT
   <213> künstliche Sequenz
<220>
   <221> PEPTIDE
   <222> (1)..(6)
   <223> Oxidoreduktase-Partialsequenz
<400> 41
   Pro Ile Lys Gly Trp Ile
   1 5
<210> 42
   <211> 6
   <212> PRT
   <213> künstliche Sequenz
<220>
   <221> PEPTIDE
   <222> (1)..(6)
   <223> Oxidoreduktase-Partialsequenz
<400> 42
   Pro Ile Ser Gly Trp Ile
   1 5